# EUROPEAN PATENT APPLICATION

(11) **EP 1 710 221 A1**
(43) Date of publication of application: **11.10.2006**
(21) Application number: 05075828.3
(22) Date of filing: 07.04.2005
(51) Int. Cl.: C07B 55/00, C12P 41/00, C07C 29/56, C07C 29/09, C07C 29/143, C07C 67/03, C07C 67/465

(54) **Process for the preparation of an optically active ester**

(71) Applicant: DSM IP Assets B.V., 6411 TE Heerlen (NL)
(72) Inventor: Heise, Andreas, 6221 JK Maastricht (NL); Verzijl, Gerardus Karel Maria, 5855 AR Well (NL); Rabani, Gouher, EH52 5DN West Lothian Scotland (GB); Palmans, Ann Rita Alberta, 6136 XG Sittard (NL)
(74) Representative: Koster, Nico

(57) **Abstract**

The invention relates to a process for the preparation of an optically active compound from raw materials comprising a di-ester and a chiral hydroxy-compound according to formula (I): 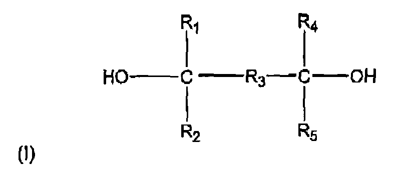 wherein:
- R₁, R₂, R₄ and R₅ each independently represent hydrogen or an alkyl group with 1-20 C-atoms, an alkenyl group with 2-20 C-atoms, an alkynyl group with 2-20 C-atoms,or an (hetero)aryl group with 4-20 C-atoms optionally containing one or more O, S or N atoms,
- R₃ represents an alkyl group with 1-20 C-atoms, an alkenyl group with 2-20 C-atoms, an alkynyl group with 2-20 C-atoms, or an (hetero)aryl group with 4-20 C-atoms optionally containing one or more O, S or N atoms,
- the compound according to formula (I) comprises at least one chiral centre that has an α-carbon relative to a secondary hydroxy group,

comprising the steps of
a) bringing the di-ester, optionally a solvent, and the chiral hydroxy-compound according to formula (I) together to form a reaction system;
b) adding an asymmetric transesterification catalyst, a racemisation catalyst and optionally a racemisation catalyst re-activating compound to the reaction system;
c) bringing the reaction system to conditions where racemisation and enantioselective transesterefication take place, whereby the optically active compound is formed,

whereby steps a), b) and c) may be executed sequentially or simultaneously, and whereby the chiral hydroxy-compound according to formula (I) may in step a) be partially or wholly replaced by the combination of the corresponding ketone and a reducing agent.

## Description

The invention relates to a process for the preparation of an optically active compound, in particular an optically active polyester.

Such a process is known from Tohru Kobayashi, Masa-aki Kakimoto, Yoshio Imai, 'Synthesis and Characterization of Optically Active Polyesters from Chiral 1,3-diols and Aromatic Dicarboxylic Acid Chlorides', Polymer Journal, Vol. 25, No. 7, pp741-746 (1993). A disadvantage of the known process is that it is necessary to first obtain a monomer in high optical purity; only then an optically active polyester can be synthesized successfully.

It is the objective of the present invention to reduce the said disadvantage.

The said objective is achieved through a process for the preparation of an optically active compound from raw materials comprising a di-ester and a chiral hydroxy-compound or mixture of compounds according to formula (I): 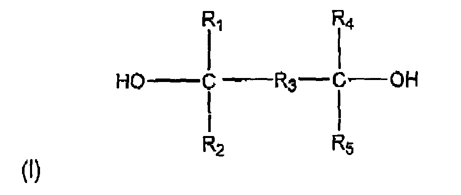 wherein:
- R₁, R₂, R₃, R₄ and R₅ each Independently represent hydrogen - whereby R₃ may not be hydrogen - or an alkyl group with for instance 1-20 C-atoms, preferably 1-6 C-atoms, an alkenyl group with for instance 2-20 C-atoms, preferably 2-6 C-atoms, an alkynyl group with for instance 2-20 C-atoms preferably 2-6 C-atoms or an (hetero)aryl group optionally containing for instance one or more O, S or N atoms, with for instance 4-20 C-atoms, preferably 5-10 C-atoms,
- the compound according to formula (I) comprises at least one chiral centre that has an α-carbon relative to a secondary hydroxy group,
comprising the steps of
a) bringing the di-ester, optionally a solvent, and the chiral hydroxy-compound according to formula (I) together to form a reaction system;
b) adding an asymmetric transesterification catalyst, a racemisation catalyst and optionally a racemisation catalyst re-activating compound to the reaction system;
c) bringing the reaction system to conditions where racemisation and enantioselective transesterefication take place, whereby the optically active compound is formed,
whereby steps a), b) and c) may be executed sequentially or simultaneously, and whereby the chiral hydroxy-compound according to formula (I) may in step a) be partially or wholly replaced by the combination of the corresponding ketone and a reducing agent.

An advantage of the process according to the invention is that it is no longer necessary to first obtain a monomer in high optical purity; instead, the optical purity of the monomer is ensured in-situ through the advantageous use of a combination of an asymmetric transesterification catalyst and a racemisation catalyst.

in the process of the invention, the raw materials from which the optically active compound is prepared comprise a di-ester. Di-esters - typically ester derivatives of dicarboxylic acids - as such are known; a wide range of di-esters are suitable for use in the process according to the invention, provided that they are suitable for transesterification.

Preferably, the di-ester is a compound or a mixture of compounds according to formula (II): 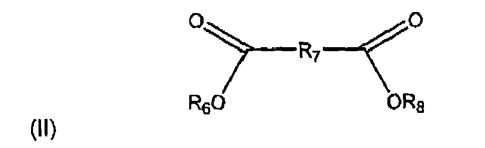 wherein R₆ and R₈ are independently from each other C₁ - C₇ of aliphatic, aromatic or cycloaliphatic nature, and where R₇ is C₂ - C₂₀ of aliphatic, aromatic or cycloaliphatic nature.

The di-ester may be prepared from a dicarboxylic acid and an alcohol. Examples of suitable dicarboxylic acid compounds from which the di-ester can be prepared include aliphatic dicarboxylic acid compounds having 2 to 20 carbon atoms such as oxalic acid, succinic acid, glutaric acid, adipic acid, pimelic acid, suberic acid, azelaic acid, sebacic acid, and dodeca diacid. Suitable aromatic dicarboxylic acids are benzenedicarboxylic acids, naphthalene dicarboxylic acids (such as naphthalene-1,4- or-1,6-dicarboxylic acid), biphenyl-x,x'-dicarboxylic acids (e.g. biphenyl-4,4'-dicarboxylic acid), diphenylacetylene-x,x'-dicarboxylic acids or stilbene-x,x'-dicarboxylic acids. Also cycloaliphatic dicarboxylic acids are suitable, such as cyclohexanedicarboxylic acids and in particular cyclohexane-1,4-dicarboxylic acid. Examples of suitable alcohols from which the di-ester can be prepared include methanol, ethanol, and (iso)propanot.

The raw materials from which the optically active compound is prepared further comprise, as indicated above, a chiral hydroxy-compound or mixture of compounds according to formula (I). Preferably, the chiral hydroxy-compound has two chiral centres that have an α-carbon relative to a secondary hydroxy group; in these compounds, one of R₁ and R₂ is H and one of R₄ and R₅ is H. Preferably, R₃ is at least C₂ or aromatic. An example of such a compound is acetyl-1,4-benzenediol.

The di-ester and the chiral hydroxy-compound are according to the invention brought together; hereby, a reaction system is formed. Optionally, a solvent may be added; this is especially useful if the raw materials have a melting point that lies above the reaction temperature. A wide range of solvents are suitable for use in the process according to the invention; care should be taken, however, that the solvent does not interfere significantly with the reaction as planned, e.g. by reacting as hydrogen donor. An example of a suitable solvent is toluene.

To the reaction system is added an asymmetric transesterification catalyst. This is understood to mean a transesterification catalyst that will preferably, predominantly or even only react with one specific stereo-configuration per chiral centre of the chiral hydroxy compound. Such enantioselective catalysts are known per se from for example Christine E Garrett et al., J.A.Chem, Soc. 120,(1998) 7479-7483 and references cited therein, and Gregory C. Fu in Chemical innovation/January 2000,3-5. Preferably the enantioselective conversion is carried out enzymatically.

Suitable enzymes that can be used in the method according to the invention are for example the known enzymes with hydrolytic activity and a high enantioselectivity in such reactions that are also active in an organic environment, for example enzymes with lipase or esterase activity or, when the di-ester is an amide, enzymes with amidase activity and esterase or lipase activity, for example originating from Pseudomonas, in particular Pseudomonas fluorescens, Pseudomonas fragi; Surkholderia, for example Burkholderia cepacia; Chromobacterium, in particular Chromobacterium viscosum; Bacillus, in particular Bacillus thermocatenulatus, Bacillus licheniformis; Alcaligenes, in particular Alcaligenes faecalis; Aspergillus, in particular Aspergillus niger; Candida, in particular Candida antarctica, Candida rugosa, Candida lipolytica, Candida cylindracea; Geotrichum, in particular Geotrichum candidum; Humicola, in particular Humicola lanuginosa; Penicillium, in particular Penicillium cyclopium, Penicillium roquefortii, Penicillium camembertii; Rhizomucor, in particular Rhizomucor javanicus, Rhizomucor miehei; Mucor, in particular Mucor javanicus; Rhizopus, in particular Rhizopus oryzae, Rhizopus arrhizus, Rhizopus delemar, Rhizopus niveus, Rhizopus Japonicus, Rhizopus javanicus; Porcine pancreas lipase, Wheat germ lipase, Bovine pancreas lipase, Pig liver esterase. Preferably an enzyme originating from Pseudomonas cepacia, Pseudomonas sp., Burkholderia cepacia, Porcine pancreas, Rhizomucor miehei, Humicola lanuginosa, Candida rugosa or Candida antarctica or subtilisin is used. If an R-selective enzyme is used, for example from Candida antarctica, the R- form of the optically active compound is obtained as product. Naturally an S-selective enzyme will lead to the S-form of the optically active compound. Such enzymes can be obtained via generally known technologies. Many enzymes are produced on a technical scale and are commercially available. The enzyme preparation as used in the present invention is not limited by purity etc. and can be both a crude enzyme solution and a purified enzyme, but it can also consist of (permeabilised and/or immobilised) cells that have the desired activity, or of a homogenate of cells with such an activity. The enzyme can also be used in an immobilised form or in a chemically modified form. The invention is in no way limited by the form in which the enzyme is used for the present invention. Within the framework of the invention it is also possible to use an enzyme originating from a genetically modified microorganism. An example of a suitable enzymatic asymmetric transesterification catalyst is Novozym® 435 (supplier: Novozymes).

To the reaction system is furthermore added a racemisation catalyst. This is understood to mean a catalyst that will reduce the enantiomeric excess of a chiral compound. Such catalysts are known as such. Examples of suitable racemisation catalysts are redox catalysts as occurring in transfer hydrogenations. The racemisation catalyst and the asymmetric transesterification catalyst are preferably chosen so that they are mutually compatible, which means that they do not or only minimally deactivate each other. The expert can establish by experimental means what asymmetric transesterification / racemisation catalyst combination is suitable for his specific system. The asymmetric transesterification catalyst and/or the racemisation can be used in heterogenised form.

Examples of racemisation catalysts to be chosen are catalysts on the basis of a transition metal compound. Transition metal compounds are described for example in Comprehensive Organometallic Chemistry 'The synthesis, Reactions and Structures of Organometallic Compounds' Volumes 1 - 9, Editor: Sir Geoffrey Wilkinson, FRS, deputy editor: F. Gordon A. Stone, FRS, Executive editor Edward W. Abel, preferably volumes 4, 5, 6 and 8 and in Comprehensive Organometallic Chemistry 'A review of the literature 1982 -1994', Editor-in-chief: Edward W. Abel, Geoffrey Wilkinson, F. Gordon A. Stone, preferably volume 4 (Scandium, Yttrium, Lanthanides and Actinides, and Titanium Group), volume 7 (Iron, Ruthenium, and Osmium), volume 8 (Cobalt, Rhodium, and Iridium), volume 9 (Nickel, Palladium, and Platinum), volume 11 (Main-group Metal Organometallics in Organic Synthesis) and volume 12 (Transition Metal Organometallics in Organic Synthesis).

As transition metal compound use is preferably made of a transition metal compound of the general formula:

MₙXₚS_{q}Lᵣ

where:
- n is 1,2,3,4....;
- p, q and r each independently represent 0,1,2,3,4...;
- M is a transition metal, for example a metal of group 7,8,9 or 10 of the periodic system according to the new IUPAC version as shown in the table printed in the cover of the Handbook of Chemistry and Physics, 70th edition, CRC press, 1989-1990, or a lanthanide or a mixture thereof, in particular iron, cobalt, nickel, rhenium, ruthenium, rhodium, iridium, osmium, palladium, platinum or samarium, or a mixture thereof. In the racemisation use is preferably made of palladium, ruthenium, iridium or rhodium, most preferably ruthenium or iridium;
- X is an anion such as e.g. hydride, halogenide, carboxylate, alkoxy, hydroxy or tetrafluoroborate;
- S is a so-called spectator ligand, a neutral ligand that is difficult to exchange, for example an aromatic compound, an olefin or a diene. Examples of aromatic compounds are: benzene, toluene, xylene, cumene, cymene, naphthalene, anisole, chlorobenzene, indene, cyclopentadienyl derivatives, tetraphenyl cyclopentadienone, dihydroindene, tetrahydronaphthalene, gallic acid, benzoic acid and phenylglycine. It is also possible for the aromatic compound to be covalently bonded to the ligand. Examples of dienes are norbomadiene, 1,5-cyclooctadiene and 1,5-hexadiene.
- L is a neutral ligand that is relatively easy to exchange with other ligands and is for example a nitrile or a co-ordinating solvent, in particular acetonitrile, dimethyl sulphoxide (DMSO), methanol, water, tetrahydrofuran, dimethyl formamide, pyridine and N-methylpyrrolidinone.

Examples of suitable transition metal compounds are: [RuCl₂(η⁶-benzene)]₂, [Rucl₂(η⁶-cymene)]₂, [RuCl₂(η⁶-mesitylene)]₂. [RuCl₂(η⁶⁻hexamethylbenzene)]₂, [RuCl₂(η⁶-1,2,3,4-tetramethylbenzene)]₂, [RuCl₂(η)⁶-1,3,5-triethylbenzene)]₂, [RuCl₂(η⁶-1,3,5-triispropylbenzene)]₂, [RuCl₂(η⁶⁻tetramethylthlophene)]₂, [RuCl₂(η⁶-methoxybenzene)]₂, [RuBr₂(η⁶-benzene)]₂, [Rul₂(η⁶⁻benzene)]₂, trans-RuCl₂(DMSO)₄, RuCl₂(PPh₃)₃, Ru3(CO)₁₂, Ru(CO)₃(η⁴ -Ph₄C₄CO), [Ru₂(CO)₄(µ-H)(C₄Ph₄COHOCC4Ph₄)], [Ir(COD)₂Cl], [Ir(CO)₂Cl]ₙ, [IrCl(CO)₃]_{n,} [Ir(Acac)(COD)], [Ir(NBD)Cl₂)₂, [Ir(COD)(C₆H₆)]⁺BF₄⁻, (CF₃C(O)CHCOCF₃)⁻[Ir(COE)₂]⁺, [Ir(CH₃CN)₄]⁺BF₄⁻, [IrCl₂Cₚ-]₂, [IrCl₂Cₚ]₂, [Rh(C₆H₁₀Cl]₂ (where C₆H₁₀ = hexa-1,5-di-ene), [RhCl₂Cₚ*]₂, (RhCl₂Cₚ]₂, [Rh(COD)Cl]₂, CoCl₂.

If necessary the transition metal compound is converted to a transition metal complex by for example exchanging the neutral ligand L with another ligand L', whereby the transition metal compound changes into MₙXₚS_{q}Lᵣ₋ₗL'ᵢ, or complexing the transition metal compound with a ligand L'. The catalyst on the basis of the transition metal compound and the ligand can be added in the form of separate components of which one is the transition metal compound and the other is the ligand L', or as a complex that contains the transition metal compound and the ligand L'. Suitable racemisation catalysts are obtained for example by complexing the transition metal compound with for example a primary or secondary amine, alcohol, diol, amino alcohol, diamine, mono-acylated diamine, mono-acylated amino alcohol, mono-tosylated diamine, mono-tosylated amino alcohol, amino acid, amino acid amide, amino-thioether, phosphine, bisphosphine, aminophosphine, preferably an aminoalcohol, monoacylated diamine, monotosyiated diamine, amino acid, amino acid amide, amino thioether or an aminophosphine. Examples of ligands are described in EP-A-916637 and in Tetrahedron: Asymmetry 10 (1999) 2045-2061, complexing not necessarily taking place with the optically active ligand, but optionally with the racemate corresponding to the optically active ligands described. The ligands are preferably used in quantities that vary between 0.5 and 8 equivalents relative to the metal, in particular between 1 and 3 equivalents. In the case of a bidentate ligand use is preferably made of 0.3-8, in particular 0.5-3 equivalents.

An example of a particularly good class of ligands is the class of amino acid amides of the formula (V). 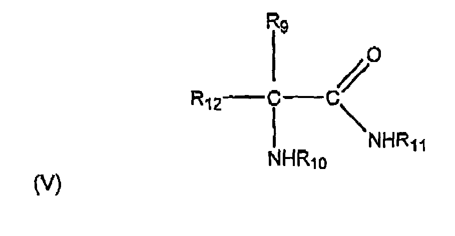 wherein R₉ and R₁₂ each independently represent H or a substituted or unsubstituted alkyl or phenyl group with for instance 1-9 C-atoms; R₁₀ and R₁₁ each independently represent H or a substituted or unsubstituted alkyl group with for instance 1-9 C-atoms, or R₉ and R₁₀ form a ring together with the N and C atom to which they are bound.

In most cases activation of catalysts, for example catalysts obtained by complexing of the transition metal compound and the ligand, can be effected for example by treating the transition metal compound or the complex of the transition metal compound and the ligand in a separate step with a base, for example KOH, KOtBu, and subsequently isolating it by removing the base. It is also possible and preferred to activate or re-activate the transition metal compound or the complex of the transition metal compound and the ligand *in situ,* when the asymmetric transesterification / racemisation takes place, with a racemisation catalyst (re)-activating compound such as a mild base, for example a heterogeneous base, in particular KHCO₃ or K₂CO₃, or a homogeneous base, in particular an organic amine, for example triethylamine. It is also possible to activate the transition metal compound with the aid of a reducing agent, for example H₂, formic acid and salts thereof, Zn and NaBH₄.

Prior to, during or subsequent to the addition of the asymmetric transesterification catalyst, the racemisation catalyst and optionally the racemisaiton catalyst re-activating compound to the reaction system, the said reaction system should be brought to conditions where both racemisation and enantioselective transesterification can take place. These conditions may be derived by the skilled person from the conditions as recommended for use of the catalysts as selected, although some routine experimentation with temperature and pressure may be necessary in order to find the correct conditions to which the reaction system should be brought. Preferably, a temperature lying between 50°C and 150°C is chosen. Additionally, the conditions should be preferably chosen such that the removal of any by-products such as R₆OH or R₈OH can be done smoothly; this can mean that the reaction system is brought to a reduced pressure, preferably to a pressure of at most 0.05, 0.02 MPa or even 0.01 MPa.

In step a), it is possible to replace the chiral hydroxy-compound according to formula (I) partly or wholly by the corresponding ketone and a reducing agent. If this is done, care should be taken to ensure that the reducing agent does not react undesirably to a significant extent with other compounds in the reaction system. For example, while it is possible to use an alcohol such as isopropanol as reducing agent, it is also know that this compound reduces the activity of certain enzymatic asymmetric transesterification catalysts.

In a preferred embodiment of the process according to the invention the chiral hydroxy-compound or mixture of compounds according to formula (I) has two chiral centres with an α-carbon relative to a secondary hydroxy group, the di-ester is a compound according to formula (II) and the asymmetric transesterification catalyst is chosen such that the optically active compound comprises or even consists essentially of a compound or mixture of compounds according to formula (III): 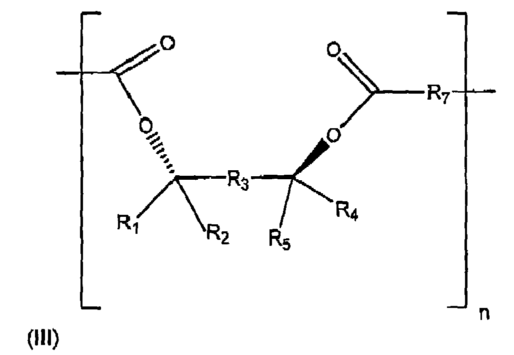 wherein R₁, R₂, R₃, R₄, R₅, and R₇ are as defined above and wherein n is an integer and at least 1. Preferably, n is at least 2, 5, 10, 15, or 20; n is preferably at most 3,000, 2,000, 1,000, 750, 500, or 350. Preferably, the asymmetric transesterification catalyst is enzymatic. As the skilled person knows, the end groups of the compound according to formula (III) can vary, a.o. depending on the molar ratio of the raw materials as used. For example, if the raw materials contain a molar excess of compound(s) according to formula (II), then the optically active compound will contain compound(s) according to formula (IIIa): 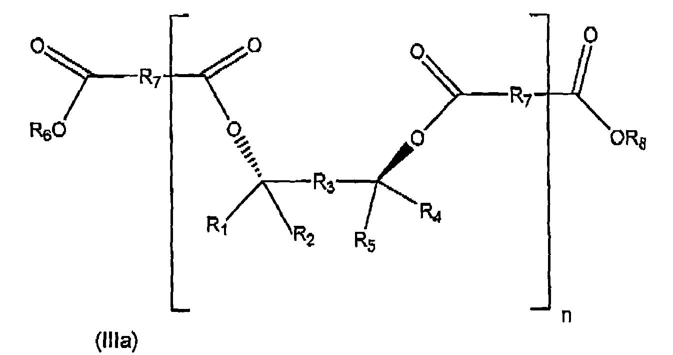 wherein R₆, and R₈ are as defined above.

Depending on the nature of the various raw materials used in the process according to the invention, it may be advantageous to implement the process according to the invention by means of an intermediate stage. In this alternative embodiment of the process according to the invention, an optically active compound is prepared from raw materials comprising a chiral hydroxy-compound according to formula (I), comprising the steps of:
d) bringing a mono-acyl donor, optionally a solvent, and a chiral hydroxy-compound according to formula (I) together to form a first reaction system;
e) adding an asymmetric transesterification catalyst, a racemisation catalyst and optionally a racemisation catalyst re-activating compound to the first reaction system;
f) bringing the first reaction system to conditions where racemisation and enantioselective transesterification take place, whereby an optically active intermediate di-ester is formed,
g) optionally isolating the optically active intermediate di-ester,
h) hydrolysing the optically active intermediate di-ester to form an optically active diol;
i) optionally isolating the optically active diol;
j) bringing the optically active diol together with a di-ester and a transesterification catalyst to form a second reaction system;
k) bringing the second reaction system to conditions where transesterification takes place, whereby the optically active compound is formed,
whereby steps d), e) and f) may be executed sequentially or simultaneously, whereby steps j) and k) may be executed sequentially or simultaneously, and whereby the chiral hydroxy-compound according to formula (I) may in step d) be partially or wholly replaced by the combination of the corresponding ketone and a hydrogen donor.

A mono-acyl donor as used in step d) is understood to mean a compound comprising one acyl group. Activated forms of mono-carboxylic acids, for example esters or amides or anhydrides, can be used as mono-acyl donor. Examples of suitable acyl donors are esters of C₁-C₂₀ mono-carboxylic acids, preferably isopropyl acetate, isopropenyl acetate, isobutyl acetate, vinyl acetate, ethyl acetate, isopropyl laureate, isopropenyl laureate or other readily available esters of mono-carboxylic acids and C₁-C₇ alcohols. Preferably a mono-acyl donor is chosen such that the mono-acyl donor itself is not significantly volatile under the reaction conditions while its mono-acyl donor residue is volatile, and oxidation of the chiral hydroxy-compound is prevented as much as possible under the reaction conditions. Examples of such mono-acyl donors are carboxylic acid esters of an alcohol with 1-4C-atoms and a carboxylic acid with 4-20C-atoms, for instance isopropyl butyric acid ester.

The other constituents of the first reaction system, namely the optional solvent, the chiral hydroxy-compound, the asymmetric transesterification catalyst, the racemisation catalyst and the optional racemisation catalyst re-activating compound, are as described above. Also, the conditions where racemisation and enantioselective transesterification take place in step f) are comparable to, if not the same as, the conditions as described for step c) above.

The optional isolating of the optically active intermediate di-ester as formed in step f) of the process according to the invention - i.e. step g) - can be done by techniques as available and known to the person skilled in the art of organic chemistry such as filtering (e.g. for separating catalyst particles from the optically active intermediate di-ester), extracting techniques such as those based on treatments with an acidic solution followed by neutralizing with a basic solution, and solvent removal techniques. In an embodiment of the isolating step g), the (preferably enzymatic) asymmetric transesterification catalyst is removed via filtering, the remaining product is extracted with an acidic solution (preferably hydrochloric acid), the organic layer is then isolated again and then neutralized with a basic solution (preferably sodium bicarbonate), the remaining product is again isolated and preferably dried with a drying agent (e.g. magnesium sulfate). Removal of the solvent in the remaining product then yields the optically active di-ester.

The steps h) and i) of hydrolysing the optically active intermediate di-ester, whereby an optically active diol is formed, and the optional isolation of the optically active diol, can be done by techniques as available and known to the person skilled in the art of organic chemistry.

In step j) of the process according to the invention, the optically active diol is brought together with a di-ester and a transesterification catalyst, whereby a second reaction system is formed. The di-ester as necessary in this step can be chosen as described above for the di-ester as used in step a) of the present invention. The transesterification catalyst as used in this step j) of the invention can be any catalyst that will achieve transesterification of the compounds as present in the second reaction system; in particular, it need not be an asymmetric transesterification catalyst as is necessary in step e).

In step k) of the process according to the invention, the second reaction system is brought to conditions where transesterification takes place, whereby the optically active compound is formed. These conditions may be derived by the skilled person from the conditions as recommended for use of the catalysts as selected, although some routine experimentation with temperature and pressure may be necessary in order to find the correct conditions to which the reaction system should be brought. Preferably, a temperature lying between 50°C and 150°C is chosen. Additionally, the conditions should be preferably chosen such that the removal of any by-products alcohols can be done smoothly; this can mean that the reaction system is brought to a reduced pressure, preferably to a pressure of at most 0.05, 0.02 MPa or even 0.01 MPa.

In a preferred embodiment of the process according to the invention, the optically active diol is a compound or a mixture of compounds according to formula (I), wherein R₂ and R₅ are H, and wherein the optically active intermediate di-ester is a compound or a mixture of compounds according to formula (IV): 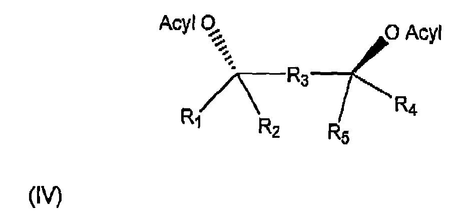 wherein R₁, R₃ and R₄ are as defined above.

The invention will be elucidated by the following Examples, without being limited thereto.

### Example 1 - formation of a racemic mixture of an optically active diol from the corresponding ketone and a hydrogen donor

To a reaction vessel (2-necked 250 ml round bottom flask fitted with a short path distillation equipment) were added:
- 1,4-Diacetylbenzene (6.10 g 37.6mmol);
- [RuClCymene]₂ (11 mg 0.018 mmol), (R,S)-a-Methylphenylglycinamide (9 mg 0.055 mmol), and potassium carbonate (0.02 g, 0.14 mmol), which together form the racemisation catalyst and racemisation catalyst re-activating compound;
- isopropanol (200ml)

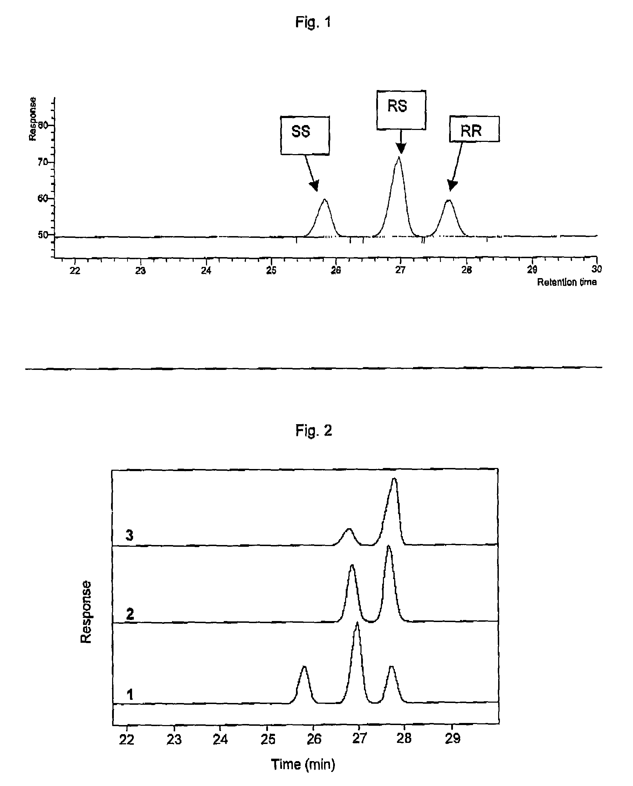

The reaction vessel was then degassed five times and the reaction system allowed to age under a nitrogen atmosphere at 75 °C for 2 hrs, after which the vacuum was gradually reduced to 0 mbar over a period of 4 hrs and the ispropanol was completely distilled off. The reaction system was then dissolved in diethylether, filtered and washed twice with 150ml of 1M HCl solution. An organic layer remained. The organic layer was then concentrated to give Acetyl-1,4-benzenediol in an isolated yield of 96%. A Gas Chromatography (GC) analysis of the Acetyl-1,4-benzenediol was done, see Figure 1. The GC showed that a racemic mixture was formed, whereby the relative peak areas were 1.57 (SS) : 3.42 (RS) : 1.56 (RR). Isolated yield (96%). Further analytical data: mp 81-82 °C; 1H-NMR(CDCl₃) : d 7.31 (s, 4H, H-Ar); 4.95 (qua, 2H, CHOH); 2.00 (s, 2H, OH); 1.50 (d, 6H, CH₃); ¹³C-NMR ¹³C-NMR (CDCl₃); d 144.9; 125.5; 69.9, 25.1. GC-MS (FW= 166.22): m/z 166 [M]⁺; 151 [M-CH₃]⁺; 133 [M-CH₃₋H₂O]⁺; 105 ; 77 [Ar]⁺; 43. Thus it can be concluded that the following reaction had taken place:

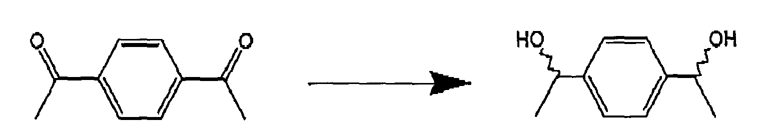

### Example 2 - Preparation of an optically active polyester from an optically active diol and a di-ester

In order to execute steps a), b) and c) according to the invention, the following actions were taken: a reaction system was formed by bringing together in a reaction vessel (a 2 necked round bottom flask fitted with a short path distillation equipment):
- the racemic mixture of acetyl-1,4-benzenediol (1.12 g, 6.74 mmol) as prepared in Example 1;
- Dimethyladipate (1.18 g, 6.74 mmol) as di-ester;
- [RuClCymen]₂ (11 mg, 0.018 mmol) and (R,S)-a-methylphenylglycinamide (9 mg, 0.054 mmol), which together form the racemisation catalyst
- Toluene (30 ml) as solvent.
- Novozym 435 (0.205 gram) as asymmetric transesterification catalyst

The reaction system was then degassed five times and then potassium carbonate (1.8 g, 13 mmol) was added as racemisation catalyst re-activating compound. The reaction system was then allowed to age at 75°C/110 mbar for 48 hrs. The reaction system was then filtered and washed twice with 100m) 1M HCl solution. An organic layer remained. The organic layer was separated and concentrated on a rotary evaporator The light yellow oil was then dried under vacuum at 80°C/8 mbar for 10 hrs to obtain the optically active polyester. GPC analysis of the polyester showed that it had a molecular weight Mw of 4000.

A chiral GC Analysis of a hydrolysed sample of the optically active polyester is given in figure 2 after 24 hours (line 2) and after 48 hours (line 3) and compared with the raw starting material (i.e. a racemic mixture of the diol, line 1). After 48 hours the relative peak areas were 0.00 (SS) : 152.22 (RS) : 642.46 (RR). This shows that an enantiomerically enriched optically active polyester was formed, as the SS variant is completely absent and the RS variant is much smaller than twice the amount of the RR variant as would be the case in a racemic mixture. The enantiomeric excess was determined to be 62%. Optical rotation measurement confirmed the chiral GC analysis, with the rotation of the un-hydrolysed polymer being [α]_{D}²⁸ = +62°.

### Example 3 - Preparation of an enantiomerically enriched optically active intermediate di-ester from a racemic mixture of an optically active diol; isolation of the optically active diol; preparation of an optically active polyester

### Execution of steps d), e), f), g) according to the invention

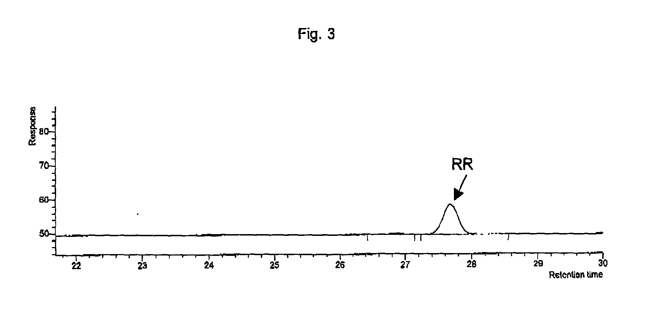

To a 2 necked 250 ml round bottom flask fitted with a short path distillation equipment were added a racemic mixture of acetyl-1,4-benzenediol (2.5 g, 15 mmol) as chiral hydroxy-compound, isopropyl butyrate (5.9 g, 45 mmol) as mono-acyl donor, [RuClCymen]₂ (11mg, 0.018mmol) as racemisation catalyst, (R,S)-α-methylphenylglycinamide (9mg, 0.054 mmol) as asymmetric transesterification catalyst and toluene (55 ml) as solvent, thereby forming the first reaction system. This first reaction system was then degassed five times after which potassium carbonate (1.8 g, 13 mmol) was added as racemisation catalyst re-activating compound. The first reaction system was then allowed to react at 75°C and 0.0110 MPa for 18 hours after which it was filtered and washed with twice with 100ml of a 1 M HCl solution. An organic layer remained. The organic layer was separated and concentrated on a rotary evaporator to yield a light yellow oil. The light yellow oil was dried under vacuum at 80°C and 0.0008 MPa for 10 hours (isolated yield 94%). Chiral GC (Figure 3) showed two peaks with the following relative areas: peak 2 (RS): 2826; peak 3 (RR): 143882, thus giving an enantiomeric excess (e.e.) of 96%.

### Execution of steps h), l) according to the invention

The isolated product from step g) according to the invention was placed into a one necked 250ml round-bottomed flask to which water (20ml), Methanol (80ml) and potassium hydroxide (3 g, 54 mmol) were added. The mixture was allowed to reflux for 24 hours. Methanol was then removed from the mixture and the remaining aqueous layer extracted three times with 100ml of ethyl acetate, which was subsequently removed by distillation to give optically pure acetyl-1,4-benzenediol (isolated yield 70%).

### Execution of steps j), k) according to the invention

To a two necked 250 ml round bottomed flask fitted with short path distillation equipment was added - in order to form the second reaction system - the optically pure acetyl-1,4-benzenediol (0.69 g, 4.2 mmol) as obtained from step i), dimethyladipate (0.72 g, 4.2 mmol) as di-ester, Novozym 435 (208mg) as transesterification catalyst and toluene (30ml) as solvent. The second reaction system was then allowed to react under reduced pressure of 0.011 MPa at 75°C, after which the Novozym was filtered off and the toluene removed by distillation, to yield the optically pure polymer. Isolated Yield 84%; optical rotation S1: [α]_{D}²⁸ = +112. The molecular weight of the optically active compound - i.e. the optically active polyester - was determined to be 6000 g mol⁻¹ as confirmed by GPC.

## Claims

1. Process for the preparation of an optically active compound from raw materials comprising a di-ester and a chiral hydroxy-compound according to formula (1): 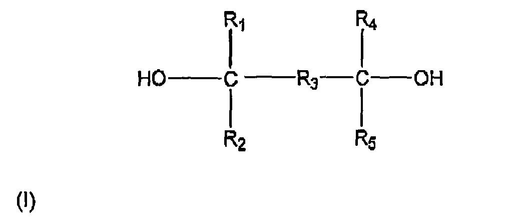 wherein:
- R₁, R₂, R₄ and R₅ each independently represent hydrogen or an alkyl group with 1-20 C-atoms, an alkenyl group with 2-20 C-atoms, an alkynyl group with 2-20 C-atoms,or an (hetero)aryl group with 4-20 C-atoms optionally containing one or more O, S or N atoms,
- R₃ represents an alkyl group with 1-20 C-atoms, an alkenyl group with 2-20 C-atoms, an alkynyl group with 2-20 C-atoms,or an (hetero)aryl group with 4-20 C-atoms optionally containing one or more O, S or N atoms,
- the compound according to formula (I) comprises at least one chiral centre that has an α-carbon relative to a secondary hydroxy group,
comprising the steps of
a) bringing the di-ester, optionally a solvent, and the chiral hydroxy-compound according to formula (I) together to form a reaction system;
b) adding an asymmetric transesterification catalyst, a racemisation catalyst and optionally a racemisation catalyst re-activating compound to the reaction system;
c) bringing the reaction system to conditions where racemisation and enantioselective transesterefication take place, whereby the optically active compound is formed,
whereby steps a), b) and c) may be executed sequentially or simultaneously, and whereby the chiral hydroxy-compound according to formula (I) may in step a) be partially or wholly replaced by the combination of the corresponding ketone and a reducing agent.

2. Process according to claim 1, wherein:
• the di-ester is a compound or a mixture of compounds according to formula (II): 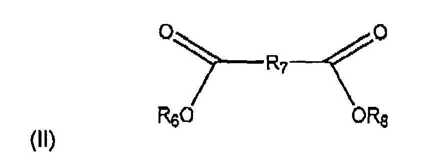 wherein R₆ and R₈ are independently from each other C₁ - C₇ of aliphatic, aromatic or cycloaliphatic nature, and where R₇ is C₂ - C₂₀ of aliphatic, aromatic or cycloaliphatic nature;
• the optically active compound comprises a compound or mixture of compounds according to formula (III): 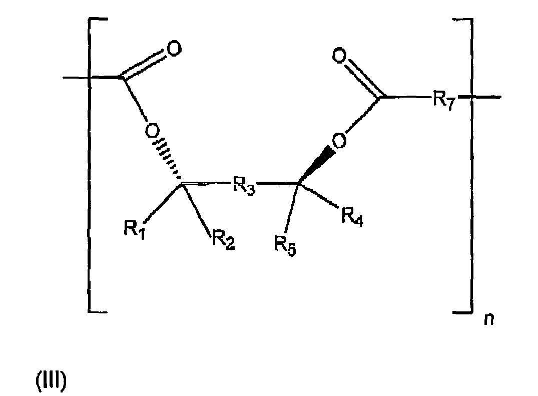 wherein R₁, R₂, R₃, R₄, R₅, and R₇ are as defined above and wherein n is an integer and at least 1.

3. Process according to claim 2, wherein the asymmetric transesterification catalyst is enzymatic, and where n is at least 5.

4. Process for the preparation of an optically active compound from raw materials comprising a chiral hydroxy-compound according to formula (I), comprising the steps of:
d) bringing a mono-acyl donor, optionally a solvent, and a chiral hydroxy-compound according to formula (I) together to form a first reaction system;
e) adding an asymmetric transesterification catalyst, a racemisation catalyst and optionally a racemisation catalyst re-activating compound to the first reaction system;
f) bringing the first reaction system to conditions where racemisation and enantioselective transesterefication take place, whereby an optically active intermediate di-ester is formed,
g) optionally isolating the optically active intermediate di-ester;
h) hydrolysing the optically active intermediate di-ester to form an optically active diol;
i) optionally isolating the optically active diol;
j) bringing the optically active diol together with a di-ester and a transesterification catalyst to form a second reaction system;
k) bringing the second reaction system to conditions where transesterefication takes place, whereby the optically active compound is formed,
whereby steps d), e) and f) may be executed sequentially or simultaneously, whereby steps j) and k) may be executed sequentially or simultaneously, and whereby the chiral hydroxy-compound according to formula (I) may in step d) be partially or wholly replaced by the combination of the corresponding ketone and a hydrogen donor.

5. Process according to claim 4, wherein the optically active diol is a compound or a mixture of compounds according to formula (I), wherein R₂ and R₅ are H, and wherein the optically active intermediate di-ester is a compound or a mixture of compounds according to formula (IV): 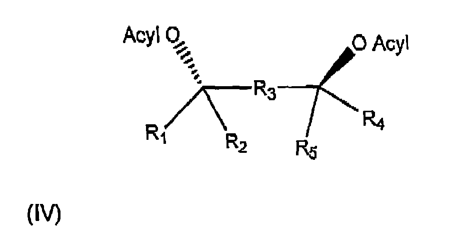 wherein R₁, R₃ and R₄ are as defined above.
